# EUROPÄISCHE PATENTSCHRIFT

(11) **EP 2 788 340 B1**
(45) Veröffentlichungstag und Bekanntmachung des Hinweises auf die Patenterteilung: **25.11.2015**
(21) Anmeldenummer: 12812161.3
(22) Anmeldetag: 07.12.2012
(51) Int. Cl.: C07D 311/94, G02B 5/23

(54) **PHOTOCHROME ZWEIFACH-INDENOANNELLIERTE NAPHTHOPYRANE**
PHOTOCHROMIC BIS-INDENOFUSED NAPHTHOPYRANS
NAPHTOPYRANES BI-INDÉNO-ANNELÉS PHOTOCHROMES

(30) Priorität: 09.12.2011 DE 102011120645
(43) Veröffentlichungstag der Anmeldung: 15.10.2014
(73) Patentinhaber: Rodenstock GmbH, 80687 München (DE)
(72) Erfinder: WEIGAND, Udo, 81247 München (DE); ZINNER, Herbert, 85296 Rohrbach (DE); ROHLFING, Yven, 81547 München (DE)
(74) Vertreter: Müller-Boré & Partner Patentanwälte PartG mbB
(86) Internationale Anmeldenummer: PCT/EP2012/005067
(87) Internationale Veröffentlichungsnummer: WO 2013/083282

(56) Entgegenhaltungen:
- WO-A1-2011/034202
- US-A- 5 645 767

## Beschreibung

Die vorliegende Erfindung betrifft photochrome zweifach-indenoannellierte Naphthopyrane der allgemeinen Formel (I) und deren Verwendung in Kunststoffen aller Art, insbesondere für ophthalmische Zwecke. Die erfindungsgemäßen photochromen Verbindungen zeichnen sich durch zwei ausgeprägte Absorptionsbanden der offenen Form im sichtbaren Wellenlängenbereich aus, d.h. mit derartigen Farbstoffmolekülen lassen sich zwei herkömmliche photochrome Farbstoffe, die jeweils nur eine diskrete Absorptionsbande aufweisen, ersetzen. Die erfindungsgemäßen Verbindungen weisen zudem eine sehr gute Lebensdauer bei sehr hoher Leistung auf.

Seit langem sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, daß diese Farbstoffmoleküle durch Lichtenergie in einen angeregten Zustand übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho[1,2-b]pyrane oder die 3,3-Diaryl-3H-naphtho[2,1-b]pyrane, die in angeregter Form verschiedene Färbungen, wie Gelb, Orange oder Rotorange, zeigen.

Als weitere Verbindungsklasse photochromer Verbindungen sind höher annellierte Pyrane von Interesse, die aufgrund ihres größeren Ringsystems längerwellig absorbieren und rote, violette und blaue Farbtöne ergeben. Diese können entweder von den 2H-Naphtho[1,2-b]pyranen oder den 3H-Naphtho[2,1-b]pyranen abgeleitete Systeme sein, die durch Annellierung an der f-Seite aus den jeweiligen Naphthopyran-Systemen hervorgehen.

2,2-Diaryl-2H-naphtho[1,2-b]pyrane mit einer zusätzlichen Annellierung am pyrano-annellierten Benzolring sind von großem Interesse, da sie aufgrund ihres größeren Ringsystems längerwellig absorbieren und so violette bzw. blaue Eindunklungsfarben zugänglich sind. Bei der Annellierung handelt es sich um einen substituierten Benzolring (in der Formel (I) der hierin beschriebenen erfindungsgemäßen Verbindungen demgemäß der Benzolring mit den Substituenten R₃), der nochmals in ortho-Stellung mit dem Naphthopyran verbrückt ist.

Wird diese Verbrückung nur über ein Atom hergestellt, so ergibt sich ein an das Naphthopyran annellierter Fünfring. Die Verwendung von Heteroatomen, insbesondere Sauerstoff, als Brücke wird in US 5,651,923 sowie US 6,018,059 beschrieben. Mit Kohlenstoff als Brückenatom ("einfach-indenoannellierte Naphthopyrane") existieren eine Reihe von Patentanmeldungen (z.B. EP 0 792 468, EP 0 906 366, EP 0 987 260, EP 1 054 010, EP 1 116 723 sowie EP 1 184 379), die sich vor allem hinsichtlich der beiden Substituenten am Kohlenstoff-Brückenatom unterscheiden. Diese Substituenten haben einen großen Einfluss auf die Aufhellgeschwindigkeit der offenen (angeregten) Form. Die offenen Formen all dieser photochromen Farbstoffe, die zusätzlich noch Substituenten wie Alkyl oder Alkoxy am nicht-indenoannellierten Benzolring der Naphthopyran-Einheit aufweisen können, weisen jeweils keine Doppelabsorptionsbande im sichtbaren Wellenlängenbereich auf. In EP 1 674 460 sowie WO 2011/034202 werden einfach-indenoannellierte Naphthopyrane offenbart, die zusätzlich einen Aryl-Substituenten am nicht-indenoannellierten Benzolring der Naphthopyran-Einheit aufweisen. In EP 0 912 908, EP 0 958 514, WO 2011/010744 sowie WO 2011/025056 werden indenoannellierte Naphthopyrane offenbart, die zusätzlich noch eine heterocyclische Annellierung am nicht-indenoannellierten Benzolring der Naphthopyran-Einheit aufweisen.

Wird diese Verbrückung über zwei Atome erzeugt, so ergibt sich ein annellierter Sechsring mit allein für C, O und N vielfältigen Möglichkeiten. Verbindungen mit C=O und N-R (Lactam-Brücke) werden in US 6,379,591 beschrieben. Verbindungen mit einer unsubstituierten CH₂-CH₂-Brücke sowie einem annellierten Heterocyclus in 7,8-Stellung des zugrundeliegenden Benzopyrans sind in US 6,426,023 offenbart. US 6,506,538 beschreibt die carbocyclischen Analogverbindungen, bei denen die H-Atome in der Brücke durch OH, (C₁ - C₆) Alkoxy oder zwei H-Atome an einem C-Atom durch =O ersetzt sein können. US 6,022,495 beschreibt *inter alia* Verbindungen mit einer O-CR¹R²-Brücke. WO 2009/024271 beschreibt analoge Verbindungen, die noch eine zusätzliche Annellierung am oberen Benzolring aufweisen.

Wird diese Verbindung durch drei Atome erzeugt, ergibt sich ein annellierter 7-Ring mit sehr vielen Variationsmöglichkeiten durch Einfügen von Heteroatomen. Verbindungen mit einer CH₂-CH₂-CH₂-Brücke sind in US 6,558,583 beschrieben. Auch hier können die H-Atome in der Brücke durch OH, (C₁ - C₆)-Alkyl oder (C₁-C₆)-Alkoxy oder zwei Wasserstoffatome an einem C-Atom durch =O ersetzt sein. Sie absorbieren bei gleichem Substitutionsmuster kürzerwellig als die annellierten 6-Ringe.

US 2004/0094753 beschreibt sowohl Verbindungen mit 2- wie mit 3-atomiger Brücke. Die zweiatomige (Kohlenstoff-)Brücke ist dabei zusätzlich mit einem Carbo- bzw. Heterocyclus annelliert. Die dreiatomige Brücke enthält drei C-Atome oder zwei C-Atome und ein O-Atom ohne zusätzliche Annellierung. Beide Ringe können vielfältige Substituenten tragen.

Die verschiedenen, im Stand der Technik verfügbaren photochromen Farbstoffe haben jedoch Nachteile, die bei der Verwendung in Sonnenschutzgläsern den Tragekomfort des Brillenträgers wesentlich beeinträchtigen. Zum einen weisen die Farbstoffe eine nicht ausreichend langwellige Absorption im angeregten wie im nicht angeregten Zustand auf. Zum anderen liegt häufig eine zu hohe Temperaturempfindlichkeit der Eindunkelung vor, wobei gleichzeitig eine zu langsame Aufhellung eintreten kann. Darüber hinaus besitzen die im Stand der Technik verfügbaren Farbstoffe oft eine ungenügende Lebensdauer und erlauben damit nur eine geringe Haltbarkeit der Sonnenschutzgläser. Letzteres macht sich in schnell nachlassender Leistung und/oder starker Vergilbung bemerkbar.

Den vorstehenden, im Stand der Technik verfügbaren photochromen Farbstoffen ist gemeinsam, dass sie nur eine Absorptionsbande der offenen Form im sichtbaren Wellenlängenbereich zeigen. Um in Neutralfarben - d.h. in Grau- oder Brauntönen - eindunkelnde phototrope Gläser zu realisieren, ist insofern ein Abstimmungsprozeß zwischen den verschiedenen photochromen Farbstoffen einer Mischung hinsichtlich Aufhellungsgeschwindigkeit, Lebensdauer sowie spektralen Anregungseigenschaften erforderlich, damit das phototrope Glas zu jedem Zeitpunkt des Eindunklungs- und Aufhellungscylus denselben Farbton aufweist. Es wäre daher äußerst wünschenswert, auf diesen Abstimmungsprozeß verzichten zu können.

Der vorliegenden Erfindung liegt daher die Aufgabe zugrunde, photochrome Farbstoffe bereitzustellen, mit denen es möglich ist, in Neutralfarben - d.h. in Grau- oder Brauntönen - eindunkelnde phototrope Gläser mit nur einem solchen photochromen Farbstoff zu realisieren. Solche photochrome Farbstoffe sollen sich zudem durch die Kombination von langwelligem Absorptionsmaximum der geschlossenen Form mit steiler Kante zum sichtbaren Wellenlängenbereich, hoher Eindunkelungsleistung, sehr schneller Aufhellreaktion und sehr guter Lichtbeständigkeit auszeichnen.

Diese Aufgabe wird durch die in den Ansprüchen gekennzeichneten Gegenstände gelöst.

Insbesondere werden photochrome zweifach-indenoannellierte Naphthopyrane mit der allgemeinen Formel (I) bereitgestellt: 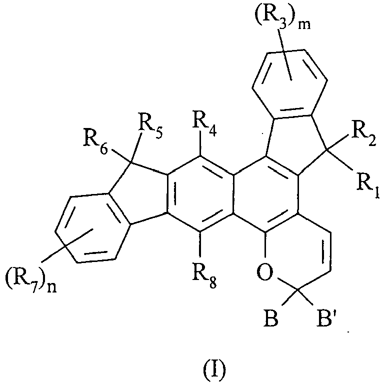 wobei die Reste R₁, R₂, R₃, R₄, R₅ ,R₆, R₇ und R₈ jeweils unabhängig voneinander einen Substituenten darstellen, ausgewählt aus der Gruppe α, bestehend aus einem Wasserstoffatom, einem (C₁-C₆)-Alkylrest, einem (C₁-C₆)-Thioalkylrest, einem (C₃-C₇)-Cycloalkylrest, der ein oder mehrere Heteroatome, wie beispielsweise O oder S, aufweisen kann, einem (C₁-C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α, vorzugsweise aus (C₁-C₆)-Alkyl, (C₁-C₆)-Alkoxy, Brom, Chlor oder Fluor, ausgewählt sein können;
m und n unabhängig voneinander eine ganze Zahl von 1 bis 4 darstellen,
oder die Reste R₁ und R₂ zusammen mit dem an diese Reste gebundenen Kohlenstoffatom einen 5- bis 7-gliedrigen carbo- oder heterocyclischen (d.h. Sauerstoff- oder Schwefelatome-haltige Heterocyclen) Ring bilden, der gegebenenfalls einen oder mehrere, vorzugsweise einen bis vier, Substituenten aus der Gruppe α trägt, wobei an diesen Ring aber auch ein bis drei aromatische oder heteroaromatische Ringsysteme annelliert sein können, wobei das bzw. die Ringsysteme unabhängig voneinander aus der Gruppe β ausgewählt ist bzw. sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, und wobei, wenn zwei dieser am 5- bis 7-gliedrigen carbo- oder heterocyclischen Ring getragenen Substituenten am selben Ringkohlenstoffatom sitzen, diese wiederum einen 5- bis 7-gliedrigen carbo- oder heterocyclischen Ring bilden können,
oder die Reste R₅ und R₆ zusammen mit dem an diese Reste gebundenen Kohlenstoffatom einen 5- bis 7-gliedrigen carbo- oder heterocyclischen (d.h. Sauerstoff- oder Schwefelatome-haltige Heterocyclen) Ring bilden, der gegebenenfalls einen oder mehrere, vorzugsweise einen bis vier, Substituenten aus der Gruppe α trägt, wobei an diesen Ring aber auch ein bis drei aromatische oder heteroaromatische Ringsysteme annelliert sein können, wobei das bzw. die Ringsysteme unabhängig voneinander aus der Gruppe β ausgewählt ist bzw. sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, und wobei, wenn zwei dieser am 5- bis 7-gliedrigen carbo- oder heterocyclischen Ring getragenen Substituenten am selben Ringkohlenstoffatom sitzen, diese wiederum einen 5- bis 7-gliedrigen carbo- oder heterocyclischen Ring bilden können,
oder zwei benachbarte Reste R₃ einen annellierten Benzol-Ring bilden, der un-, mono- oder disubstituiert sein kann, wobei die Substituenten wiederum aus der Gruppe α ausgewählt sein können;
oder zwei benachbarte Reste R₇ einen annellierten Benzol-Ring bilden, der un-, mono- oder disubstituiert sein kann, wobei die Substituenten wiederum aus der Gruppe α ausgewählt sein können;
und B und B' unabhängig voneinander aus einer der folgenden Gruppen a) oder b) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist,
wobei die Substituenten der Aryl- oder Heteroarylreste in a) und b) solche sind, ausgewählt aus der vorstehend definierten Gruppe α oder der Gruppe χ, bestehend aus Amino, Mono-(C₁-C₆)-alkylamino, Di-(C₁-C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]lazepinyl, wobei der oder die Substituenten unabhängig voneinander wiederum aus (C₁-C₆)-Alkyl, (C₁-C₆)-Alkoxy, Brom, Chlor oder Fluor ausgewählt sein können;
oder wobei zwei direkt benachbarte Substituenten der Aryl- oder Heteroarylreste in a) und b) eine V-(CR₈R₉)ₚ-W-Gruppierung darstellen, wobei p = 1, 2 oder 3 ist, die Reste R₈ und R₉ jeweils unabhängig voneinander einen Substituenten darstellen, ausgewählt aus der Gruppe α, und wobei V und W unabhängig voneinander -O-, -S-, -N(C₁-C₆)Alkyl, -NC₆H₅, -CH₂-, -C(CH₃)₂- oder -C(C₆H₅)₂- sein können, wobei zwei oder mehrere benachbarte CR₈R₉-Einheiten dieser V-(CR₈R₉)ₚ-W-Gruppierung Teil eines daran annellierten Benzolrings sein können, welcher jeweils wiederum einen oder mehrere Substituenten, ausgewählt aus der Gruppe α aufweisen kann, oder V und/oder W zusammen mit der jeweils benachbarten CR₈R₉ Einheit einen annellierten Benzolring, der un-, mono- oder disubstituiert sein kann, wobei dessen Substituenten aus der Gruppe α ausgewählt sein können, darstellen.

Die erfindungsgemäßen Verbindungen zeichnen sich gegenüber den im Stand der Technik (US 5,645,767) bekannten photochromen 2H-Naphtho[1,2-b]pyranen, die keine zweite Indeno-Annellierung aufweisen, dadurch aus, dass sie eine Doppelabsorptionsbande, d.h. zwei Banden, der offenen Form im sichtbaren Wellenlängenbereich zeigen, wenn eine zweite Indeno-Annellierung eingeführt ist. Die erste der beiden starken Absorptionsbanden weist dabei ein Absorptionsmaximum von > 500 nm auf, während das Maximum der zweiten Bande im kürzerwelligen sichtbaren Bereich (400-500 nm) liegt. Aufgrund letzterer Bande ist es mit den erfindungsgemäßen Verbindungen möglich, auf gelb- oder orange-eindunkelnde photochrome Farbstoffe in neutralfarbenen phototropen Gläsern zu verzichten. Dies ist einerseits wichtig für Polymersysteme, in denen diese gelb- und orange-eindunkelnden Farbstoffe - aufgrund ihrer anderen Molekülstruktur im Vergleich zu den längerwellig absorbierenden violett- und blaueindunkelnden Farbstoffen - eine ungenügende Lebensdauer aufweisen oder andere Nachteile mit sich bringen. Andererseits ist es mit den erfindungsgemäßen photochromen Farbstoffen erstmals möglich, in Neutralfaben - d.h. in Grau- oder Brauntönen - eindunkelnde phototrope Gläser mit nur einem photochromen Farbstoff zu realisieren. Damit entfällt der bisher notwendige mühsame Abstimmungsprozess zwischen den verschiedenen photochromen Farbstoffen einer Mischung hinsichtlich Aufhellungsgeschwindigkeit, Lebensdauer sowie spektralen Anregungseigenschaften, damit das phototrope Glas zu jedem Zeitpunkt des Eindunklungs- und Aufhellungscylus denselben Farbton aufweist.

Da die erfindungsgemäßen Verbindungen darüber hinaus hohe Augenklarheit (d.h. hohe Transmission im nicht angeregten Zustand) sowie sehr gute Lichtbeständigkeit aufweisen, sind sie hervorragend geeignet zum Einsatz in phototropen Gläsern.
Fig. 1 zeigt ein entsprechendes Syntheseschema zur Herstellung der erfindungsgemäßen Verbindungen.
Fig. 2 zeigt die UV-Absorptionsspektren spezifischer erfindungsgemäßer Verbindung im Vergleich zum Stand der Technik.

In einer Ausführungsform der vorliegenden Erfindung sind die Reste R₁ und R₂ unabhängig voneinander aus einem Wasserstoffatom, einem (C₁-C₆)-Alkylrest oder einem (C₃-C₇)-Cycloalkylrest, vorzugsweise einem (C₁-C₆)-Alkylrest oder einem (C₃-C₇)-Cycloalkylrest, ausgewählt.

In einer anderen Ausführungsform der vorliegenden Erfindung bilden die Reste R₁ und R₂ zusammen mit dem an diese Reste gebundenen Kohlenstoffatom einen 5-bis 7-gliedrigen carbo- oder heterocyclischen Ring, der gegebenenfalls einen oder mehrere Substituenten aus der Gruppe α trägt.

In einer weiteren Ausführungsform der vorliegenden Erfindung sind die Reste R₅ und R₆ unabhängig voneinander aus einem Wasserstoffatom, einem (C₁-C₆)-Alkylrest oder einem (C₃-C₇)-Cycloalkylrest, vorzugsweise einem (C₁-C₆)-Alkylrest, ausgewählt.

Bevorzugte photochrome zweifach-indenoannellierte Naphthopyrane gemäß der vorliegenden Erfindung weisen die nachfolgende allgemeine Formeln (II) auf: 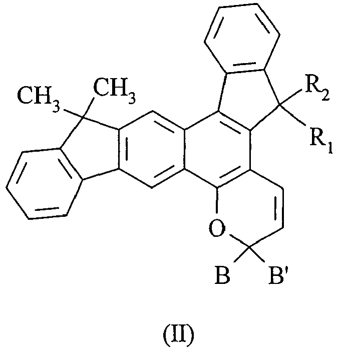 wobei die Reste R₁, R₂, B sowie B' wie vorstehend definiert sind.

In einer weiteren bevorzugten Ausführungsform sind die Reste B und B' unabhängig voneinander aus der Gruppe a), wie vorstehend definiert, ausgewählt.

Die Substituenten der Gruppe χ, welche Stickstoffatome aufweisen bzw. Amingruppen tragen, sind über diese an den Phenyl-, Naphthyl- bzw. Phenanthrylrest der Gruppe a) gebunden.

Wenn bezüglich der Substituenten der Gruppe V-(CR₈R₉)ₚ-W-Gruppierung, welche an den Phenyl, Naphthyl- bzw. Phenanthrylrest der Gruppe a) für die Reste B bzw. B' gebunden sein können, zwei oder mehrere benachbarte Kohlenstoffatome dieser V-(CR₈R₉)ₚ-W-Gruppierung jeweils unabhängig voneinander Teil eines daran annellierten Benzo-Ringsystems sind, so bedeutet dies, dass dann die beiden Methylenkohlenstoffatome (-CH₂-CH₂-) Teil eines annellierten Ringsystems werden. Wenn beispielsweise zwei oder drei Benzoringe annelliert sind, so können beispielsweise hier dann folgende Struktureinheiten vorliegen, wie nachstehend angeführt.

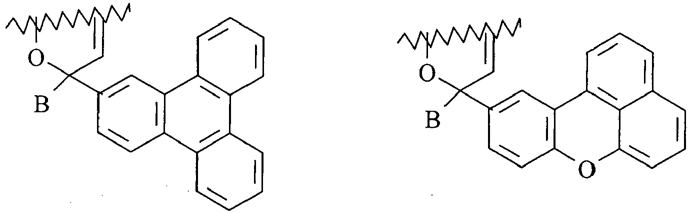

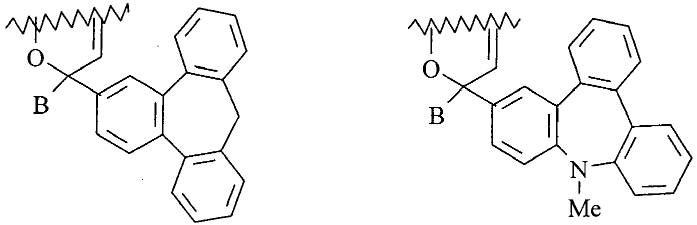

Selbstverständlich kann aber auch nur ein, über zwei benachbarte Kohlenstoffatome dieser V-(CR₈R₉)ₚ-W-Gruppierung annellierter Benzoring vorliegen.

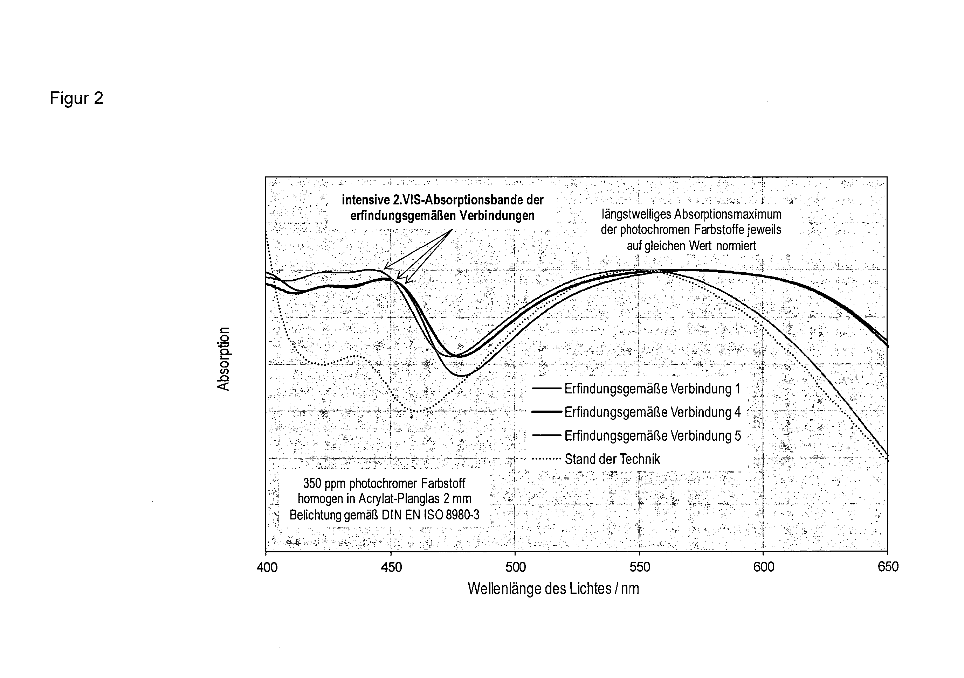

Wie bereits ausgeführt, weisen die erfindungsgemäßen Verbindungen gegenüber den im Stand der Technik (US 5,645,767) bekannten photochromen 2H-Naphtho[1,2-b]pyranen, die keine zweite Indeno-Annellierung aufweisen, überraschenderweise eine zweite starke Absorptionsbande der offenen Form im sichtbaren Wellenlängenbereich auf (siehe Figur 2). Die Ausbildung dieser zweiten Absorptionsbande bei den erfindungsgemäßen Verbindungen ist insofern unerwartet.

Zur Messung der spektralen Eigenschaften der erfindungsgemäßen Verbindungen wurden jeweils 350 ppm des photochromen Farbstoffes in einer Acrylatmonomer-Matrix gelöst und nach Zusatz eines Polymerisations-Initiators mit Hilfe eines Temperaturprogrammes thermisch polymerisiert. Die Transmissionseigenschaften im angeregten Zustand der so hergestellten Kunststoffgläser (Dicke 2mm) wurden anschließend gemäß DIN EN ISO 8980-3 gemessen.

Die Strukturen der in Figur 2 eingesetzten bzw. untersuchten Verbindungen sind aus der folgenden Tabelle ersichtlich:

**Tabelle 1: Tabellarischer Vergleich der längstwelligen Absorptionsmaxima im angeregten Zustand (An = Anisyl, d.h. der 4-Methoxyphenyl-Rest)**

| | | | | | |
|---|---|---|---|---|---|
| 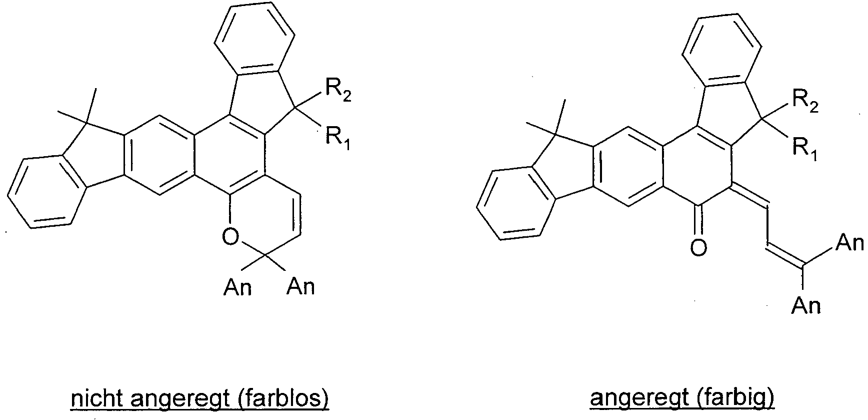 | | | | | |

| | R₁ | R₂ | λmax (1) | λmax (2) | Farbeindruck |
|---|---|---|---|---|---|
| Stand der Technik aus US 5,645,767 (ohne den erfindungsgemäßen zweiten annellierten Indeno-Ring) | Methyl | Methyl | - | 550 nm | Violett |
| Erfindungsgemäße Verbindung 1 | Methyl | Methyl | 450 nm | 575 nm | Blaugrünliches Grau |
| Erfindungsgemäße Verbindung 2 | Ethyl | Ethyl | 450 nm | 575 nm | Blaugrünliches Grau |
| Erfindungsgemäße Verbindung 3 | Propyl | Propyl | 450 nm | 575 nm | Blaugrünliches Grau |
| Erfindungsgemäße Verbindung 4 | -(CH₂)₅-(Spiro-Ring) | | 450 nm | 575 nm | Bläuliches Grau |
| Erfindungsgemäße Verbindung 5 | Cyclohexyl | Cyclohexyl | 440 nm | 550 nm | Rotviolett |

Fig. 2 zeigt die UV-Absorptionsspektren der erfindungsgemäßen Verbindungen 1, 4 und 5 im Vergleich zum Stand der Technik. Die Ausbildung einer Doppelabsorptionsbande bei der erfindungsgemäßen Verbindung 1 im Gegensatz zum Stand der Technik zeigt deutlich den Einfluss der zweiten Indeno-Annellierung auf das Absorptionsspektrum - bei ansonsten gleicher Molekülstruktur (siehe Figur 2). Die erfindungsgemäßen Verbindungen 2 und 3 weisen eine zu Verbindung 1 identisches Absorptionsverhalten auf.

Auffallend ist die hypsochrome Verschiebung beider Absorptionsbanden der erfindungsgemäßen Verbindung 5 im Vergleich zu den anderen erfindungsgemäßen Verbindungen. Außerdem weist diese Verbindung auch bei der quantitativen Messung der Eindunklungstiefe eine deutlich geringere Sättigungsabsorption auf (siehe Tabelle 2). Da die Verbindung laut HPLC/NMR rein ist, lassen sich die beobachteten Unterschiede zu den anderen erfindungsgemäßen Verbindungen allein durch die höhere sterische Belastung durch die beiden sehr sperrigen Cyclohexyl-Substituenten erklären. Dadurch wird der obere Benzolring aus der Molekülebene herausgedreht und dessen Konjugation mit dem Restmolekül behindert. Dies führt dann zur beobachteten hypsochromen Verschiebung der Absorptionsbanden und Verringerung der erreichbaren Eindunklungstiefe.

Die Tabelle 2 zeigt einen Überblick über die Eindunklungstiefe im angeregten Zustand sowie die Aufhellgeschwindigkeit der erfindungsgemäßen Verbindungen (Formeln analog Tabelle 1; Messung an 2 mm dicken Kunststoffgläsern gemäß DIN EN ISO 8980-3 jeweils bei 23°C).

**Tabelle 2: Photochrome Eigenschaften der erfindungsgemäßen Verbindungen**

| Erfindungsgemäße Verbindung | 4 | 1 | 2 | 3 | 5 |
|---|---|---|---|---|---|
| Sättigungsabsorption bei 23°C (angeregter Zustand) | 95% | 90% | 88% | 85% | 78% |
| Erreichte Aufhellung nach 2 Minuten bei 23°C * | 4 rel% | 17 rel% | 28 rel% | 27 rel% | 15 rel% |
| Erreichte Aufhellung nach 10 Minuten bei 23°C * | 25 rel% | 59 rel% | 71 rel% | 69 rel% | 52 rel% |

| | | | | | |
|---|---|---|---|---|---|
| * angegeben in Relation zum Ausgangszustand: 100 rel% würde vollständige Aufhellung zum Ausgangszustand bedeuten (je höher der Wert; umso schneller ist die Aufhellgeschwindigkeit) | | | | | |

Die erfindungsgemäßen Verbindungen sind in Tabelle 2 von links nach rechts nach zunehmender Größe der Substituenten R₁ und R₂ angegeben. Die erfindungsgemäße Verbindung 4 weist die geringste von R₁ und R₂ ausgehende sterische Belastung auf (beide bilden zusammen einen Spiro-Sechsring) und die höchste beobachtete Sättigungsabsorption (Eindunklungstiefe). Die Aufhellgeschwindigkeit ist jedoch mit Abstand die langsamste in der Reihe. Die erfindungsgemäße Verbindung 5 weist die größte von R₁ und R₂ ausgehende sterische Belastung auf (zwei sehr sperrige Cyclohexyl-Reste).

Normalerweise ist zu erwarten, dass aufgrund des photochromen Reaktionsmechanismus (Molekülöffnung in die angeregte Form durch UV-Licht; thermische Rückreaktion in die nicht angeregte Form) die Aufhellgeschwindigkeit bei konstanter Temperatur indirekt proportional zur Sättigungsabsorption ist, d.h. je tiefer eine photochrome Verbindung eindunkelt, umso langsamer hellt sie normalerweise auch auf. In der Reihe in Tabelle 2 ist jedoch interessanterweise ein Maximum der Aufhellgeschwindigkeit zu erkennen. Der Grund hierfür liegt in der bereits beschriebenen starken sterischen Belastung der erfindungsgemäßen Verbindung 5 (und mit Abstrichen auch der Verbindung 3), die eine Verdrillung des Moleküls zur Folge hat und damit einhergehend eine geringere Sättgungsabsorption.

Die erfindungsgemäße Verbindung 2 weist sowohl eine recht tiefe Sättigungsabsorption als auch eine recht schnelle Aufhellgeschwindigkeit auf.

Tabelle 2 zeigt, dass die photochromen Eigenschaften durch der Auswahl der Substituenten R₁ und R₂ maßgeschneidert werden können. Sämtliche Verbindungen weisen aber den gewünschten Doppelabsorptionscharakter in der angeregten Form auf.

Durch die vorliegende Erfindung wird eine Klasse neuer photochromer Doppelabsorptionsfarbstoffe bereitgestellt, die sowohl Verbindungen mit extrem tiefer Eindunklung und langsamer Aufhellgeschwindigkeit (für phototrope Outdoor-Produkte und höhere Temperaturen) als auch Verbindungen mit schnellerer Aufhellgeschwindigkeit (für phototrope Alltagsgläser) enthält.

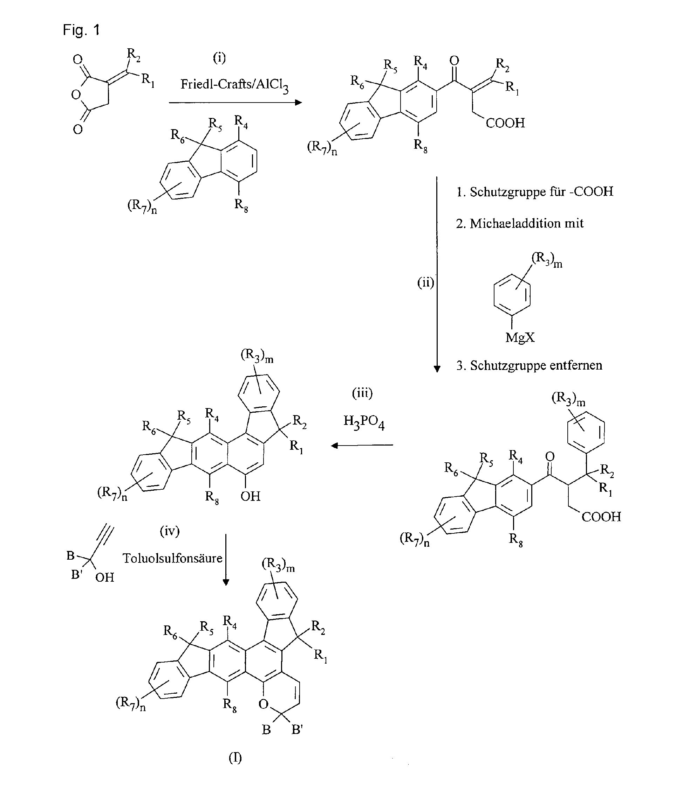

Zur Synthese der erfindungsgemäßen Verbindungen werden geeignet substituierte Methylidensuccinanhydride in einem ersten Schritt einer Friedel-Crafts-Reaktion mit geeignet substituierten Fluoren-Derivaten unterworfen (Schritt (i)). Die -COOH Gruppe des daraus resultierenden Intermediats wird anschließend geschützt und dieses Intermediat einer Michael-Addition mit entsprechend substituierten Aryl-Grignardverbindungen unterworfen (Schritt (ii)). Nach Entfernung der Carbonsäure-Schutzgruppe werden *via* intramolekularer Cyclisierung mittels Phosphorsäure entsprechend substituierte Derivate gebildet (Schritt (iii)). Anschließend werden diese substituierten Derivate mit geeignet substituierten 2-Propin-1-ol-Derivaten gemäß Schritt (iv) zu den erfindungsgemäßen Verbindungen umgesetzt. Das vorstehende Syntheseschema ist in Figur 1 wiedergegeben.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen zweifach-indenoannellierten Naphthopyran-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen und dergleichen eingesetzt werden. Ferner können die erfindungsgemäßen photochromen zweifach-indenoannellierten Naphthopyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen zweifach-indenoannellierten Naphthopyrane durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen zweifach-indenoannellierten Naphthopyrane beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

## Patentansprüche

1. Photochrome zweifach-indenoannellierte Naphthopyrane mit der allgemeinen Formel (I): 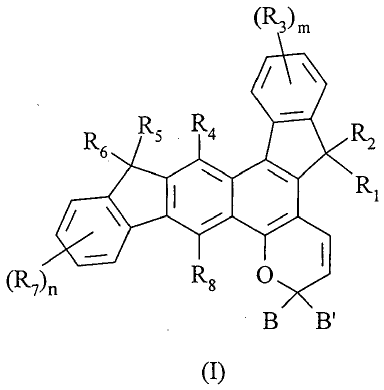 wobei die Reste R₁, R₂, R₃, R₄, R₅ ,R₆, R₇ und R₈ jeweils unabhängig voneinander einen Substituenten darstellen, ausgewählt aus der Gruppe α, bestehend aus einem Wasserstoffatom, einem (C₁-C₆)-Alkylrest, einem (C₁-C₆)-Thioalkylrest, einem (C₃-C₇)-Cycloalkylrest, der ein oder mehrere Heteroatome aufweisen kann, einem (C₁-C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α, vorzugsweise aus (C₁-C₆)-Alkyl, (C₁-C₆)-Alkoxy, Brom, Chlor oder Fluor, ausgewählt sein können;
m und n unabhängig voneinander eine ganze Zahl von 1 bis 4 darstellen,
oder die Reste R₁ und R₂ zusammen mit dem an diese Reste gebundenen Kohlenstoffatom einen 5- bis 7-gliedrigen carbo- oder heterocyclischen Ring bilden, der gegebenenfalls einen oder mehrere Substituenten aus der Gruppe α trägt, wobei an diesen Ring aber auch ein bis drei aromatische oder heteroaromatische Ringsysteme annelliert sein können, wobei das bzw. die Ringsysteme unabhängig voneinander aus der Gruppe β ausgewählt ist bzw. sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, und wobei, wenn zwei dieser am 5- bis 7-gliedrigen carbo- oder heterocyclischen Ring getragenen Substituenten am selben Ringkohlenstoffatom sitzen, diese wiederum einen 5- bis 7-gliedrigen carbo- oder heterocyclischen Ring bilden können,
oder die Reste R₅ und R₆ zusammen mit dem an diese Reste gebundenen Kohlenstoffatom einen 5- bis 7-gliedrigen carbo- oder heterocyclischen Ring bilden, der gegebenenfalls einen oder mehrere Substituenten aus der Gruppe α trägt, wobei an diesen Ring aber auch ein bis drei aromatische oder heteroaromatische Ringsysteme annelliert sein können, wobei das bzw. die Ringsysteme unabhängig voneinander aus der Gruppe β ausgewählt ist bzw. sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, und wobei, wenn zwei dieser am 5- bis 7-gliedrigen carbo- oder heterocyclischen Ring getragenen Substituenten am selben Ringkohlenstoffatom sitzen, diese wiederum einen 5- bis 7-gliedrigen carbo- oder heterocyclischen Ring bilden können,
oder zwei benachbarte Reste R₃ einen annellierten Benzol-Ring bilden, der un-, mono- oder disubstituiert sein kann, wobei die Substituenten wiederum aus der Gruppe α ausgewählt sein können;
oder zwei benachbarte Reste R₇ einen annellierten Benzol-Ring bilden, der un-, mono- oder disubstituiert sein kann, wobei die Substituenten wiederum aus der Gruppe α ausgewählt sein können;
und B und B' unabhängig voneinander aus einer der folgenden Gruppen a) oder b) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist,
wobei die Substituenten der Aryl- oder Heteroarylreste in a) und b) solche sind, ausgewählt aus der vorstehend definierten Gruppe α oder der Gruppe χ, bestehend aus Amino, Mono-(C₁-C₆)-alkylamino, Di-(C₁-C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁-C₆)-Alkyl, (C₁-C₆)-Alkoxy, Brom, Chlor oder Fluor ausgewählt sein können;
oder wobei zwei direkt benachbarte Substituenten der Aryl- oder Heteroarylreste in a) und b) eine V-(CR₈R₉)ₚ-W-Gruppierung darstellen, wobei p = 1, 2 oder 3 ist, die Reste R₈ und R₉ jeweils unabhängig voneinander einen Substituenten darstellen, ausgewählt aus der Gruppe α, und wobei V und W unabhängig voneinander -O-, -S-, -N(C₁-C₆)Alkyl, -NC₆H₅, -CH₂-, -C(CH₃)₂- oder -C(C₆H₅)₂- sein können, wobei zwei oder mehrere benachbarte CR₈R₉-Einheiten dieser V-(CR₈R₉)ₚ-W-Gruppierung Teil eines daran annellierten Benzolrings sein können, welcher jeweils wiederum einen oder mehrere Substituenten, ausgewählt aus der Gruppe α aufweisen kann, oder V und/oder W zusammen mit der jeweils benachbarten CR₈R₉ Einheit einen annellierten Benzolring, der un-, mono- oder disubstituiert sein kann, wobei dessen Substituenten aus der Gruppe α ausgewählt sein können, darstellen.

2. Photochrome zweifach-indenoannellierte Naphthopyrane gemäß Anspruch 1, wobei die Reste R₁ und R₂ unabhängig voneinander aus einem Wasserstoffatom, einem (C₁-C₆)-Alkylrest oder einem (C₃-C₇)-Cycloalkylrest, vorzugsweise einem (C₁-C₆)-Alkylrest oder einem (C₃-C₇)-Cycloalkylrest, ausgewählt sind.

3. Photochrome zweifach-indenoannellierte Naphthopyrane gemäß Anspruch 1, wobei R₁ und R₂ zusammen mit dem an diese Reste gebundenen Kohlenstoffatom einen 5- bis 7-gliedrigen carbo- oder heterocyclischen Ring bilden, der gegebenenfalls einen oder mehrere Substituenten aus der Gruppe α trägt.

4. Photochrome zweifach-indenoannellierte Naphthopyrane gemäß einem der Ansprüche 1 bis 3, wobei R₅ und R₆ unabhängig voneinander aus einem Wasserstoffatom, einem (C₁-C₆)-Alkylrest oder einem (C₃-C₇)-Cycloalkylrest ausgewählt sind.

5. Photochrome zweifach-indenoannellierte Naphthopyrane gemäß einem der Ansprüche 1 bis 4, welche die nachfolgende allgemeine Formel (II) aufweisen: 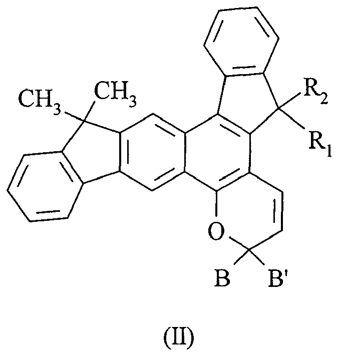 wobei die Reste R₁, R₂, B sowie B' wie vorstehend definiert sind.

6. Photochrome zweifach-indenoannellierte Naphthopyrane gemäß einem der Ansprüche 1 bis 5, wobei die Reste B und B' unabhängig voneinander aus der Gruppe a), wie vorstehend definiert, ausgewählt sind.

7. Verwendung der photochromen Naphthopyrane nach einem der Ansprüche 1 bis 6 in und auf Kunststoffmaterialien.

8. Verwendung nach Anspruch 7, wobei das Kunststoffmaterial eine ophthalmische Linse ist.

## Claims

1. Photochromic, bis-indenofused naphthopyrans with the general Formula (I): 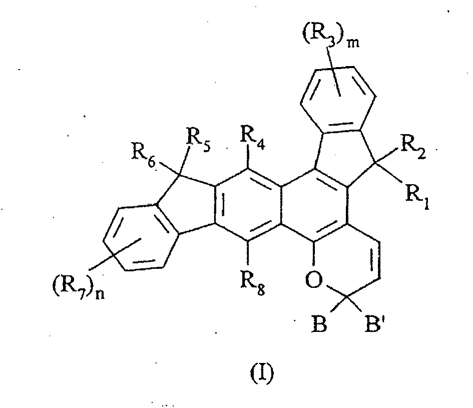 wherein the radicals R₁, R₂, R₃, R₄, R₅, R₆, R₇ and R₈, respectively independently of each other, represent a substituent selected from the group α consisting of a hydrogen atom, a (C₁-C₆) alkyl radical, a (C₁-C₆) thioalkyl radical, a (C₃-C₇) cycloalkyl radical, which can have one or more heteratoms, a (C₁-C₆) alkoxy radical, a hydroxy group, a trifluoromethyl group, bromine, chlorine, fluorine, an un-, mono- or disubstituted phenyl-, phenoxy-, benzyl-, benzyloxy-, naphthyl- or naphthoxy radical, the substituents in turn being able to be selected from the group α, preferably from (C₁-C₆) alkyl, (C₁-C₆) alkoxy, bromine, chlorine or fluorine;
m und n, independently of each other, representing a whole number from 1 to 4,
or the radicals R₁ and R₂, together with the carbon atom bonded to these radicals, form a 5- to 7-membered carbo- or heterocyclic ring, which possibly carries one or more substituents from the group α, but also one to three aromatic or heteroaromatic ring systems being able to be fused on this ring, the ring system(s), independently of each other, being selected from the group β, consisting of benzene, naphthalene, phenanthrene, pyridine, quinoline, furan, thiophene, pyrrol, benzofuran, benzothiophene, indole and carbazole, which in turn can be substituted with one or more substituents, selected from the group α, and if two of these substituents carried on the 5- to 7-membered carbo- or heterocyclic ring are situated on the same ring carbon atom, these in turn being able to form a 5- to 7-membered carbo- or heterocyclic ring,
or the radicals R₅ and R₆, together with the carbon atom bonded to these radicals, form a 5- to 7-membered carbo- or heterocyclic ring, which possibly carries one or more substituents from the group α, but also one to three aromatic or heteroaromatic ring systems being able to be fused on this ring, the ring system(s), independently of each other, being selected from the group β, consisting of benzene, naphthalene, phenanthrene, pyridine, quinoline, furan, thiophene, pyrrol, benzofuran, benzothiophene, indole and carbazole, which in turn can be substituted with one or more substituents, selected from the group α, and if two of these substituents carried on the 5- to 7-membered carbo- or heterocyclic ring are situated on the same ring carbon atom, these in turn being able to form a 5- to 7-membered carbo- or heterocyclic ring,
or two adjacent radicals R₃ form a fused benzene ring, which can be un-, mono- or disubstituted, the substituents in turn being able to be selected from the group α;
or two adjacent radicals R₇ form a fused benzene ring, which can be un-, mono- or disubstituted, the substituents in turn being able to be selected from the group α;
and B and B', independently of each other, are selected from the following groups a) or b),
a) being mono-, di- and trisubstituted aryl radicals, the aryl radical being phenyl, naphthyl or phenanthryl;
b) being unsubstituted, mono- and disubstituted heteroaryl radicals, the heteroaryl radical being pyridyl, furanyl, benzofuranyl, thienyl, benzothienyl, 1,2,3,4-tetrahydrocarbazolyl or julolidinyl,
the substituents of the aryl- or heteroaryl radicals in a) and b) being those selected from the previously defined group α or from the group x, consisting of amino, mono-(C₁-C₆) alkylamino, di-(C₁-C₆) alkylamino, on the phenyl ring, un-, mono- or disubstituted mono- and diphenylamino, piperidinyl, N-substituted piperazinyl, pyrrolidinyl, imidazolidinyl, pyrazolidinyl, indolinyl, morpholinyl, 2,6-dimethylmorpholinyl, thiomorpholinyl, azacycloheptyl, azacyclooctyl, un-, mono- or disubstituted phenothiazinyl, un-, mono- or disubstituted phenoxazinyl, un-, mono- or disubstituted 1,2,3,4-tetrahydroquinolinyl, un-, mono- or disubstituted 2,3-dihydro-1,4-benzoxazinyl, un-, mono- or disubstituted 1,2,3,4-tetrahydroisoquinolinyl, un-, mono- or disubstituted phenazinyl, un, mono- or disubstituted carbazolyl, un-, mono- or disubstituted 1,2,3,4-tetrahydrocarbazolyl and un-, mono- or disubstituted 10,11-dihydrodibenzo[b,f]azepinyl, the substituent(s), independently of each other, in turn being able to be selected from (C₁-C₆) alkyl, (C₁-C₆) alkoxy, bromine, chlorine or fluorine;
or two directly adjacent substituents of the aryl- or heteroaryl radicals in a) and b) representing a V-(CR₈R₉)ₚ-W grouping, p being = 1, 2 or 3, the radicals R₈ and R₉, respectively independently of each other, representing a substituent selected from the group α, and V and W, independently of each other, being able to be -O-,-S-, -N(C₁-C₆) alkyl, -NC₆H₅, -CH₂-, -C(CH₃)₂- or C(C₆H₅)₂-, two or more adjacent CR₈R₉ units of this V-(CR₈R₉)ₚ-W grouping being able to be part of a benzene ring fused thereon, which respectively in turn can have one or more substituents selected from the group α, or V and/or W, together with the respectively adjacent CR₈R₉ unit, representing a fused benzene ring, which can be un-, mono- or disubstituted, the substituents thereof being able to be selected from the group α.

2. Photochromic, bis-indenofused naphthopyrans according to claim 1, the radicals R₁ and R₂, independently of each other, being selected from a hydrogen atom, a (C₁-C₆) alkyl radical or a (C₃-C₇) cycloalkyl radical, preferably a (C₁-C₆) alkyl radical or a (C₃-C₇) cycloalkyl radical.

3. Photochromic, bis-indenofused naphthopyrans according to claim 1, R₁ and R₂, together with the carbon atom bonded to these radicals, forming a 5- to 7-membered carbo- or heterocyclic ring, which possibly carries one or more substituents from the group α.

4. Photochromic, bis-indenofused naphthopyrans according to one of the claims 1 to 3, R₅ and R₆, independently of each other, being selected from a hydrogen atom, a (C₁-C₆) alkyl radical or a (C₃-C₇) cycloalkyl radical.

5. Photochromic, bis-indenofused naphthopyrans according to one of the claims 1 to 4, which have the subsequent general Formula (II): 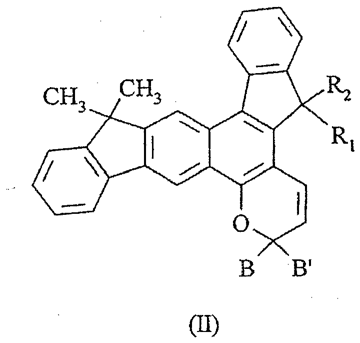 the radicals R₁, R₂, B and also B' being as previously defined.

6. Photochromic, bis-indenofused naphthopyrans according to one of the claims 1 to 5, the radicals B and B', independently of each other, being selected from the group a) as previously defined.

7. Use of the photochromic naphthopyrans according to one of the claims 1 to 6 in and on plastic materials.

8. Use according to claim 7, the plastic material being an ophthalmic lens.

## Revendications

1. Naphtopyranes bi-indéno-annelés photochromes de formule générale (I) : 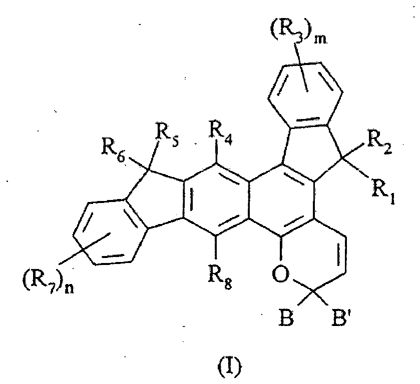 où les radicaux R₁, R₂, R₃, R₄, R₅, R₆, R₇ et R₈ représentent respectivement, indépendamment les uns des autres, un substituant choisi dans le groupe α, consistant en un atome d'hydrogène, un radical alkyle (en C₁ à C₆), un radical thioalkyle (en C₁ à C₆), un radical cycloalkyle (en C₃ à C₇) qui peut présenter un ou plusieurs hétéroatomes, un radical alcoxy (en C₁ à C₆), un groupe hydroxy, un groupe trifluorométhyle, un atome de brome, de chlore, de fluor, un radical phényle, phénoxy, benzyle, benzyloxy, naphtyle ou naphtoxy non substitué, monosubstitué ou disubstitué, les substituants pouvant être choisis eux-mêmes dans le groupe α, de préférence parmi les groupes alkyle (en C₁ à C₆), alcoxy (en C₁ à C₆), un atome de brome, de chlore ou de fluor ;
m et n représentent, indépendamment l'un de l'autre, un nombre entier de 1 à 4,
ou les radicaux R₁ et R₂ forment, conjointement avec l'atome de carbone lié à ces radicaux, un cycle carbocyclique ou hétérocyclique de 5 à 7 chaînons qui porte éventuellement un ou plusieurs substituants du groupe α, où sur ce cycle peuvent toutefois être également annelés un à trois systèmes cycliques aromatiques ou hétéro-aromatiques, où le ou les système(s) cyclique(s) est ou sont choisi(s), indépendamment les uns des autres, dans le groupe P consistant en les groupes benzène, naphtaline, phénanthrène, pyridine quinoline, furane, thiophène, pyrrole, benzofurane, benzothiophène, indole et carbazole qui peuvent eux-mêmes être substitués par un ou plusieurs substituants choisis dans le groupe α, et où, si deux de ces substituants portés sur le cycle carbocyclique ou hétérocyclique de 5 à 7 chaînons se trouvent sur le même atome de carbone cyclique, ceux-ci peuvent former eux-mêmes un cycle carbocyclique ou hétérocyclique de 5 à 7 chaînons,
ou les radicaux R₅ et R₆ forment, conjointement avec l'atome de carbone lié à ces radicaux, un cycle carbocyclique ou hétérocyclique de 5 à 7 chaînons qui porte éventuellement un ou plusieurs substituants du groupe α, où sur ce cycle peuvent toutefois être également annelés un à trois systèmes cycliques aromatiques ou hétéro-aromatiques, où le ou les système(s) cyclique(s) est ou sont choisi(s), indépendamment les uns des autres, dans le groupe β consistant en les groupes benzène, naphtaline, phénanthrène, pyridine quinoline, furane, thiophène, pyrrole, benzofurane, benzothiophène, indole et carbazole qui peuvent eux-mêmes être substitués par un ou plusieurs substituants choisis dans le groupe α, et où, si deux de ces substituants portés sur le cycle carbocyclique ou hétérocyclique de 5 à 7 chaînons se trouvent sur le même atome de carbone cyclique, ceux-ci peuvent former eux-mêmes un cycle carbocyclique ou hétérocyclique de 5 à 7 chaînons,
ou deux radicaux R₃ voisins forment un cycle benzène annelé qui peut être non substitué, monosubstitué ou disubstitué, les substituants pouvant être choisis eux-mêmes dans le groupe α ;
ou deux radicaux R₇ voisins forment un cycle benzène annelé qui peut être non substitué, monosubstitué ou disubstitué, les substituants pouvant être choisis eux-mêmes dans le groupe α ;
et B et B' sont choisis, indépendamment l'un de l'autre, dans l'un des groupes a) ou b) suivants, où
a) se trouvent des radicaux aryle monosubstitués, disubstitués et trisubstitués, le radical aryle étant un groupe phényle, naphtyle ou pénanthryle ;
b) se trouvent des radicaux hétéroaryle non substitués, monosubstitués et disubstitués, le radical hétéroaryle étant un groupe pyridyle, furanyle, benzofuranyle, thiényle, benzothiényle, 1,2,3,4-tétrahydrocarbazolyle ou julolidinyle,
où les substituants des radicaux aryle ou hétéroaryle dans les groupes a) et b) sont ceux qui sont choisis dans le groupe α défini précédemment ou dans le groupe χ consistant en les groupes amino, mono-alkylamino (en C₁ à C₆), di-alkylamino (en C₁ à C₆), monophénylamino et diphénylamino non substitués, monosubstitués ou disubstitués sur le cycle phényle, pipéridinyle, pipérazinyle N-substitué, pyrrolidinyle, imidazolidinyle, pyrazolidinyle, indolinyle, morpholinyle, 2,6-diméthylmorpholinyle, thiomorpholinyle, azacycloheptyle, azacyclooctyle, phénothiazinyle non substitué, monosubstitué ou disubstitué, phénoxazinyle non substitué, monosubstitué ou disubstitué, 1,2,3,4-tétrahydroquinolinyle non substitué, monosubstitué ou disubstitué, 2,3-dihydro-1,4-benzoxazinyle non substitué, monosubstitué ou disubstitué, 1,2,3,4-tétrahydro-isoquinolinyle non substitué, monosubstitué ou disubstitué, phénazinyle non substitué, monosubstitué ou disubstitué, carbazolyle non substitué, monosubstitué ou disubstitué, 1,2,3,4-tétrahydrocarbazolyle non substitué, monosubstitué ou disubstitué et 10,11-dihydrodibenz[b,f]azépinyle non substitué, monosubstitué ou disubstitué, où le ou les substituants peuvent être eux-mêmes choisis, indépendamment les uns des autres, parmi les groupes alkyle (en C₁ à C₆), alcoxy (en C₁ à C₆), un atome de brome, de chlore ou de fluor ;
ou où deux substituants directement voisins des radicaux aryle ou hétéroaryle dans les groupes a) et b) représentent un groupe V- (CR₈R₉)ₚ-W, où p = 1, 2 ou 3, les radicaux R₈ et R₉ représentent respectivement, indépendamment l'un de l'autre, un substituant choisi dans le groupe α, et où V et W peuvent être, indépendamment l'un de l'autre, -O-, -S-, un groupe -N-alkyle (en C₁ à C₆), -NC₆H₅, -CH₂-, -C(CH₃)₂- ou -C(C₆H₅)₂, où deux ou plusieurs motifs CR₈R₉ voisins de ce groupe V-(CR₈R₉)ₚ-W peuvent être une partie d'un cycle benzène annelé à celui-ci, qui peut lui-même respectivement présenter un ou plusieurs substituants choisis dans le groupe α, ou V et/ou W, conjointement avec le motif CR₈R₉ respectivement voisin, peuvent représenter un cycle benzène annelé qui peut être non substitué, monosubstitué ou disubstitué, ses substituants pouvant être choisis dans le groupe α.

2. Naphtopyranes bi-indéno-annelés photochromes selon la revendication 1, où les radicaux R₁ et R₂ sont choisis, indépendamment l'un de l'autre, parmi un atome d'hydrogène, un radical alkyle (en C₁ à C₆) ou un radical cycloalkyle (en C₃ à C₇), de préférence un radical alkyle (en C₁ à C₆) ou un radical cycloalkyle (en C₃ à C₇).

3. Naphtopyranes bi-indéno-annelés photochromes selon la revendication 1, où les radicaux R₁ et R₂ forment, conjointement avec l'atome de carbone lié à ces radicaux, un cycle carbocyclique ou hétérocyclique de 5 à 7 chaînons qui porte éventuellement un ou plusieurs substituants du groupe α.

4. Naphtopyranes bi-indéno-annelés photochromes selon l'une des revendications 1 à 3, où R₅ et R₆ sont choisis, indépendamment l'un de l'autre, parmi un atome d'hydrogène, un radical alkyle (en C₁ à C₆) ou un radical cycloalkyle (en C₃ à C₇).

5. Naphtopyranes bi-indéno-annelés photochromes selon l'une des revendications 1 à 4, qui présentent la formule générale (II) suivante : 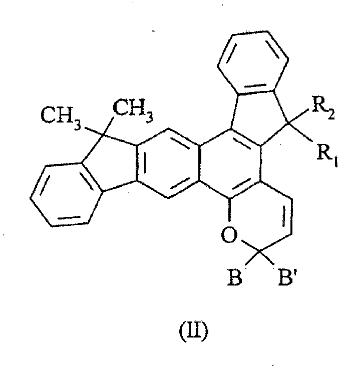 où les radicaux R₁, R₂, B et B' sont tels que définis précédemment.

6. Naphtopyranes bi-indéno-annelés photochromes selon l'une des revendications 1 à 5, où B et B' sont choisis, indépendamment l'un de l'autre, dans le groupe a) tel que défini précédemment.

7. Utilisation des napthopyranes photochromes selon l'une des revendications 1 à 6, dans et sur des matériaux en plastique.

8. Utilisation selon la revendication 7, où le matériau en plastique est une lentille ophtalmique.
